# FASCICULE DE BREVET EUROPEEN

(11) **EP 3 781 081 B1**
(45) Date de publication et mention de la délivrance du brevet: **13.09.2023**
(21) Numéro de dépôt: 19724556.6
(22) Date de dépôt: 18.04.2019
(51) Int. Cl.: A61F 2/01

(54) **FILTRE DE TYPE PARAPLUIE AVEC PROTECTION ANTI-TRANSFIXION**
SCHIRMFILTER MIT ANTI-DURCHSTICHSCHUTZ
UMBRELLA TYPE FILTER WITH ANTI-TRANSFIXION PROTECTION

(30) Priorité: 18.04.2018 FR 1853412
(43) Date de publication de la demande: 24.02.2021
(73) Titulaire: A.L.N., 20240 Ghisonaccia (FR)
(72) Inventeur: NIGON, Alain, 83230 Bormes les Mimosas (FR)
(74) Mandataire: Plasseraud IP
(86) Numéro de dépôt international: PCT/FR2019/050934
(87) Numéro de publication internationale: WO 2019/202274

(56) Documents cités:
- EP-A1- 2 997 933
- WO-A1-2011/055079
- US-A1- 2006 015 137
- US-A1- 2008 033 534
- US-A1- 2010 121 373

## Description

### Domaine technique

La présente invention concerne le domaine technique des dispositifs médicaux et plus particulièrement des filtres pour vaisseaux sanguins et notamment des filtres caves.

### État de la technique

Afin de prévenir les conséquences parfois fatales des thromboses veineuses profondes migrantes, des filtres peuvent être disposé à l'intérieur de la veine cave et notamment dans sa partie inférieure. En effet, ces thromboses se forment généralement sur la paroi des veines au niveau des membres inférieurs avant de se détacher et de remonter vers le coeur par la veine cave inférieure. Si les thromboses atteignent les poumons, elles peuvent venir obstruer les artères pulmonaires conduisant à une embolie pulmonaire qui peut s'avérer fatale.

Les filtres caves permettent d'arrêter la thrombose avant qu'elle n'atteigne les poumons en créant un obstacle stoppant l'avancée de celle-ci.

Un exemple de filtre cave est un filtre cave de type parapluie. Ce type de filtre comprend généralement des branches, chacune comportant une extrémité fixe et une extrémité libre. Les extrémités fixes des branches étant fixées les unes aux autres, notamment par sertissage à une base, généralement de forme cylindrique à extrémité arrondie. Les branches, dites branches d'ancrage, s'étendent donc à partir de la base en s'écartant les unes des autres telles les baleines d'un parapluie. Ainsi, les branches d'ancrage confèrent au filtre une enveloppe extérieure ayant la forme d'un cône ou d'un clocher à bulbe. En d'autres termes, cette enveloppe est formée par la révolution autour d'un axe d'une ligne droite coupant cet axe ou d'une ligne courbe naissant à un point proche de l'axe et s'étendant en s'éloignant de celui-ci.

Pour être efficaces, ces filtres caves de type parapluie doivent être ancrés à la paroi de la veine cave. Pour cela, les extrémités libres se terminent en crochet orienté vers l'extérieur. Ces crochets vont s'ancrer dans la paroi de la veine cave pour fixer la position du filtre.

Malheureusement, dans certains cas, les branches d'ancrage transpercent la paroi de la veine cave provoquant la détérioration d'un organe adjacent pouvant entrainer un saignement interne qui peut avoir de sérieuses conséquences pour le patient. Notamment, dans une revue rétrospective, « Retrospective Review of 120 Celect Inferior Vena Cava Filter Retrievals: Expérience at a Single Institution » (« Revue rétrospective de 120 retraits du filtre pour veine cave inférieure Celect : expérience dans une seule institution » en français), in J Vase Interv Radiol 2012 ; 23:1557-1563, Zhou D.Y. et son équipe ont pu observer un taux de transfixion de 86,1 % chez les 115 patients s'étant présenté pour le retrait du filtre et pour lesquels un cavogram par projection antéropostérior était disponible et de 65,8 % chez les 38 patients s'étant présenté pour le retrait du filtre et pour lesquels une image par tomodensitométrie était disponible dont 9 chez qui la transfixion a atteint un organe adjacent au site de pose du filtre. Les auteurs ont conclu que le filtre utilisé présentait un taux de transfixion élevé corrélé à la durée pendant laquelle le filtre est demeuré dans la veine cave inférieure, bien que ces chiffres ne prennent pas en compte les patients chez qui le filtre était posé et qui ne se sont pas présentés pour un retrait du filtre.

Par ailleurs, il est important que ces filtres caves de type parapluie soient bien alignés par rapport à l'axe local de la veine cave, c'est-à-dire que la direction de l'axe de révolution de l'enveloppe de ces filtres caves de type parapluie ne doit pas trop différer de celle de l'axe local de la veine cave. Si l'inclinaison du filtre dépasse un certain degré, l'efficacité du filtre peut être diminuée.

Par exemple, dans une étude du filtre Günther Tulip : « Analysis of Tilt of the Günther Tulip Filter » (« Analyse de l'inclinaison du filtre Günther Tulip » en français), in J Vase Interv Radiol 2008 ; 19:669-676, Sag A.A. et son équipe ont observé que chez 159 patients parmi les 175 qui se sont présentés pour un retrait du filtre présentent une inclinaison du filtre dont le degré d'inclinaison moyen est 7,1° ± 5,8.

EP2997933A1 divulgue un filtre pour veine cave.

### Présentation de l'invention

Ainsi, un des objectifs de la présente invention est de remédier à au moins un des désavantages des filtres de l'état de la technique mentionnés ci-dessus.

L'invention concerne un filtre pour veine cave selon l'objet de la revendication indépendante 1.

Grâce à la protection anti-transfixion, tout transpercement de la paroi du vaisseau sanguin dans lequel est disposé le filtre est évité.

D'autres caractéristiques optionnelles et non-limitatives du filtre sont présentés ci-après.

La protection anti-transfixion est une vrille réalisée sur la branche d'ancrage à proximité du crochet. La protection anti-transfixion est une vrille présentant un diamètre équivalent compris entre 990 et 1210 µm. Selon des configurations non couvertes par l'invention, la protection anti-transfixion peut être un zig-zag ou une ondulation réalisée sur la branche d'ancrage à proximité du crochet.

Au moins deux branches d'ancrage peuvent présenter des longueurs différentes.

Le filtre peut comprendre en outre trois branches de centrage courtes, chacune comprenant une extrémité fixe et une extrémité libre, l'extrémité fixe étant fixée aux extrémités fixes des branches d'ancrage, et l'extrémité libre étant atraumatique. Les trois branches de centrage courtes peuvent être plus courtes que les branches d'ancrage. L'extrémité libre atraumatique peut présenter un crochet orienté vers l'intérieur. Les branches de centrage courtes peuvent être de longueur égale ou de longueurs différentes.

Le filtre peut comprendre en outre trois branches de centrage longues, chacune présentant une extrémité fixe et une extrémité libre, l'extrémité fixe étant fixée aux extrémités fixes des branches d'ancrage. Les trois branches de centrage longues peuvent être plus longues que les branches d'ancrage.

Le filtre peut comprendre en outre une branche de contact présentant une extrémité fixe et une extrémité libre, l'extrémité fixe étant fixée aux extrémités fixes des branches d'ancrage et l'extrémité libre comprenant un butoir. Le butoir peut être une boucle réalisée à partir de la branche de contact.

L'extrémité libre d'une des branches de centrage longues peut comprendre un butoir. Le butoir peut être une boucle réalisée à partir de la branche de centrage longue.

Le filtre peut être entièrement réalisé en acier inoxydable, notamment INOX 316 LVM.

Un autre aspect de la divulgation non couvert par l'invention propose également un filtre pour vaisseau sanguin, et notamment filtre cave, comprenant au moins deux branches d'ancrage pour l'ancrage du filtre dans la paroi du vaisseau sanguin, les branches d'ancrage comportant une extrémité fixe et une extrémité libre, les extrémités fixes des branches d'ancrage étant fixée l'une à l'autre et terminée en crochet orienté vers l'extérieur ; et trois branches de centrage plus courtes que les branches d'ancrage, chacune comprenant une extrémité fixe et une extrémité libre, l'extrémité fixe étant fixées aux extrémités fixes des branches d'ancrage, et l'extrémité libre étant atraumatique.

Grâce à ces branches de centrage plus courtes que les branches d'encrage, l'inclinaison du filtre par rapport à sa position idéale peut être limitée.

### Dessins

D'autres objectifs, caractéristiques et avantages apparaitront à la lecture de la description illustrative qui suit en référence aux dessins donnés à titre non limitatif et à titre d'exemple, parmi lesquels :
- la figure 1 est une illustration en trois-quarts d'un exemple de filtre selon l'invention ;
- la figure 2 est un agrandissement de la protection anti-transfixion pour le filtre de la figure 1. Les configurations de protection anti-transfixion des fig. 3-5 ne sont pas couvertes par l'invention;
- la figure 6 est une illustration d'un adaptateur pour l'insertion du filtre de la figure 1 à l'intérieur d'un cathéter.

### Description

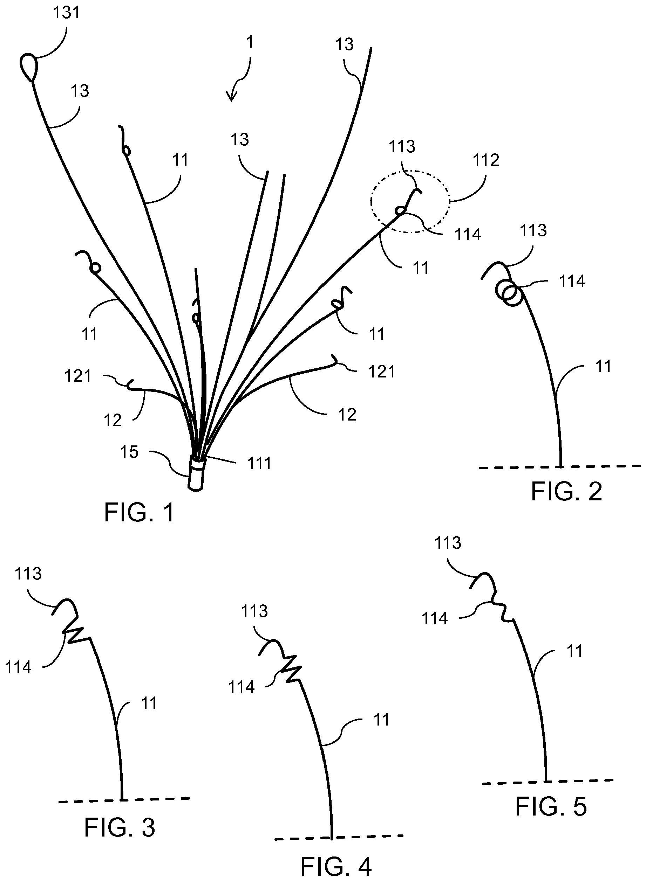

Un filtre pour vaisseau sanguin, notamment un filtre cave, de type parapluie selon l'invention sera décrit ci-après en référence aux figures 1 à 5.

On entend par filtre **1** de type parapluie, des filtres comportant une pluralité de branches **11, 12, 13,** chaque branche ayant une extrémité fixe et une extrémité libre. Les extrémités fixes des branches sont fixées les unes aux autres par l'intermédiaire d'une pièce **15** dont la forme est généralement une sphère, un fuseau, un cylindre à base circulaire, une ogive, etc., les arrêtes étant préférentiellement arrondies. La fixation est généralement effectuée par sertissage avec ou sans soudage laser. Les branches **11, 12, 13** s'écartent progressivement à partir du point de fixation les unes des autres. Chacune des branches **11, 12, 13** présente généralement une section droite circulaire. Les branches **11, 12, 13** s'étendent indépendamment les unes des autres, une ligne droite, une ligne convexe, une ligne concave, une ligne présentant un point de flexion présentant une première portion plus proche de l'extrémité fixe concave et une deuxième portion plus proche de l'extrémité libre convexe, une ligne présentant un point de flexion présentant une première portion plus proche de l'extrémité fixe convexe et une deuxième portion plus proche de l'extrémité libre concave, etc. La concavité des lignes est considérée d'un point de vue situé dans l'espace ouvert formé par les branches, c'est-à-dire à l'intérieur du parapluie. Le filtre comprend généralement un axe longitudinal. L'axe longitudinal est la moyenne d'axes d'extension des branches, l'axe d'extension d'une branche étant un axe reliant ses extrémités. Ainsi, de préférence, si l'on considère deux points de la ligne formée par une branche, le point le plus proche de l'extrémité fixe n'est pas plus éloigné de l'axe longitudinal que le point le plus proche de l'extrémité libre. De préférence toute branche peut décrire une ligne s'inscrivant dans un plan, avantageusement intersectant l'axe longitudinal du filtre. La description de la forme des branches ci-dessus, ne prend pas en compte les crochets et protection anti-transfixion, notamment ceux décrits ci-dessous.

Le filtre **1** de l'invention comprend au moins deux branches d'ancrage **11** (par exemple 2, 3, 4, 5, 6, 7, 8, 9, 10 branches d'ancrage) pour l'ancrage du filtre **1** dans la paroi du vaisseau sanguin, les branches d'ancrage **11** comportant une extrémité fixe **111** et une extrémité libre **112.** Le filtre **1** de l'invention comprend de préférence au moins 3, au moins 4, au moins 5, au moins 6, au moins 7, au moins 8, au moins 9 ou au moins 10 branches d'ancrage **11.**

Les extrémités fixes **111** des branches d'ancrage sont fixées les unes aux autres par la pièce **15** et les extrémités libres **112** sont terminées par un crochet **113** orienté vers l'extérieur du filtre afin de permettre l'ancrage du filtre **1** à la paroi du vaisseau sanguin. Avantageusement, le crochet **113** présente un diamètre équivalent compris entre 400 et 1100 µm, de préférence entre 750 et 950. Dans le présent exposé, le terme « diamètre équivalent » d'un crochet désigne le diamètre du cercle le plus petit dans lequel peut s'inscrire le crochet, de son extrémité jusqu'au premier minimum local de rayon de courbure, celui-ci pouvant être nul.

Chacune des branches d'ancrage **11** comprend en outre une protection anti-transfixion **114.** La protection anti-transfixion **114** comprend une vrille dessinée par la branche d'ancrage elle-même à proximité du crochet **113** (voir figures 1 et 2). Dans le présent exposé, le terme de « vrille » est compris comme désignant la forme que réalise une branche dont la ligne décrit à proximité du crochet une courbe revenant sur elle-même. À l'endroit où la courbe revient sur elle-même, les deux parties de la branche peuvent être en contact ou non, de préférence, elles sont en contact.

La vrille présente un diamètre équivalent compris entre 990 et 1210 µm, de préférence entre 1100 µm et 1150 µm. Dans le présent exposé, le terme « diamètre équivalent » d'une vrille désigne le diamètre du cercle le plus petit dans lequel peut s'inscrire la projection de la boucle sur un plan d'aire intérieure la plus grande. De préférence, la forme de la projection de la boucle sur un plan d'aire d'intérieur la plus grande est un cercle.

Les vrilles de deux branches différentes peuvent être de diamètres différents. Elles peuvent être alternativement de même diamètre. De préférence, les vrilles de toutes les branches sont de même diamètre avec une tolérance de ± 10 %. Toujours de préférence, les vrilles de toutes les branches sont des cercles.

Les vrilles de deux branches différentes peuvent présenter des formes différentes. Elles peuvent être alternativement de même forme.

Le nombre de vrilles peut être augmenté, par exemple au moins 2, au moins 3 ou au moins 4. Lorsqu'il y a plus d'une vrille, les vrilles peuvent être disposées soit les unes adjacentes aux autres ou les unes en chevauchement total ou partiel avec les autres. Dans ce cas, en outre des possibilités décrites ci-dessus, toutes les vrilles d'une même branche peuvent être de diamètres différents. Elles peuvent être alternativement de même diamètre. De préférence, les vrilles de toutes les branches sont de même diamètre avec une tolérance de ± 10 %. Toujours de préférence, les vrilles de toutes les branches sont des cercles. En outre, elles peuvent présenter des formes différentes. Elles peuvent être alternativement de même forme.

D'autres formes de protection anti-transfixion, non couvertes par l'invention, sont également possibles, par exemple :
- la forme zigzag (dents de scie), notamment présentant 1, 2, 3 périodes ou plus, et/ou dont l'angle des sommets est compris entre 30 et 90°, par exemple 40°, 45°, 50°, 60°, 70°, 80°, et/ou la largeur crête-à-crête est avantageusement comprise 990 et 1210 µm, de préférence entre 1100 µm et 1150 µm (voir figures 3 et 4) ; et
- une forme ondulée, notamment présentant 1, 2, 3 périodes ou plus, et/ou la largeur crête-à-crête est avantageusement comprise 990 et 1210 µm, de préférence entre 1100 µm et 1150 µm et/ou une longueur de période comprise entre 1 et 5 mm (voir figure 5).
Ces formes sont disposées comme la vrille à proximité du crochet. La taille de ces protection anti-transfixion permet notamment d'éviter l'endothélisation du filtre pour un retrait sûr et moins traumatique pour le patient.

Au moins deux branches d'ancrage **11** peuvent présenter des longueurs différentes. Ceci permet d'éviter que leurs crochets ne s'accrochent entre eux lors du déploiement du filtre comme décrit ci-dessous. De préférence, les branches d'ancrage présentent des longueurs différentes les unes des autres.

La ligne décrite par chaque branche d'ancrage **11** peut présenter l'une des formes décrites ci-dessus. De préférence, les lignes décrites par les branches d'ancrage **11** sont convexes.

Le filtre peut comprendre en outre au moins trois branches de centrage courtes **12** (par exemple 3, 4, 5, 6 branches de centrage courtes). La ligne décrite par chaque branche de centrage courte **12** peut présenter l'une des formes décrites ci-dessus. De préférence, les lignes décrites par les branches de centrage courtes **12** sont convexes. Les branches de centrage courtes **12** sont de préférence plus courtes que les branches d'ancrage **11.** De préférence de 35 à 55 %, toujours de préférence 40 à 50 %, encore de préférence environ 45 %, plus courtes que la branche d'ancrage la plus courte. Les branches de centrage courtes **12** peuvent être de longueurs différentes ou égales. De préférence, elles sont de longueurs égales.

Chaque branche de centrage courte **12** présente une extrémité fixe et une extrémité libre, l'extrémité fixe étant fixée aux extrémités fixes des branches d'ancrage notamment par la pièce **15,** et l'extrémité libre étant atraumatique.

Le caractère atraumatique de l'extrémité libre peut être conféré par un crochet **121** courbe orienté vers l'intérieur, notamment quand la ligne de la branche de centrage courte **12** est convexe. Ainsi la courbe formée par la branche au niveau du crochet est orientée vers la paroi du vaisseau sanguin lorsque le filtre est en place, évitant de blesser celle-ci. Avantageusement, le crochet **121** présente un diamètre équivalent compris entre 1500 et 1850 µm, de préférence entre 1650 et 1750 µm.

En variante, le caractère atraumatique de l'extrémité libre peut être conféré par la ligne de la branche de centrage courte **12** qui présente alors un point d'inflexion et une portion partant de l'extrémité libre sensiblement colinéaire à l'axe longitudinal du filtre.

Le filtre peut encore comprend au moins trois branches de centrage longues **13,** de préférence au moins 4, au moins 5. Le qualificatif « longues » est à mettre en regard du qualificatif « courtes » des branches de centrage courtes **12.** De préférence, les trois branches de centrage longues **13** sont plus longues que les branches d'ancrage (sur la figure 1, l'une des branches de centrage longues parait plus courte à cause de la perspective). De préférence 20 à 35 %, toujours de préférence 25 à 30 %, encore de préférence environ 27 %, plus longues que la branche d'ancrage **11** la plus longue. Les branches de centrage longues **13** peuvent être de longueurs différentes ou égales. De préférence, elles sont de longueurs égales notamment quand elles se terminent en crochets orientés vers l'intérieur du filtre.

Chacune des branches de centrage longues **13** présente une extrémité fixe et une extrémité libre, l'extrémité fixe étant fixée aux extrémités fixes des branches d'ancrage **11,** notamment via la pièce **15.**

La ligne décrite par chaque branche de centrage longue **13** peut présenter l'une des formes décrites ci-dessus. De préférence, les lignes décrites par les branches de centrage longues **13** présentent un point d'inflexion avec une partie convexe entre l'extrémité fixe et le point d'inflexion et une partie concave entre le point d'inflexion et l'extrémité libre.

Avantageusement, l'extrémité libre d'au moins une des branches de centrage longues **13** comprend un butoir **131.** Le butoir **131** permet de pousser le filtre **1,** notamment par voie fémorale, lors de son placement à l'intérieur du vaisseau sanguin. Alternativement, le filtre **1** comprend en outre une branche de contact présentant une extrémité fixe et une extrémité libre, l'extrémité fixe étant fixée aux extrémités fixes des branches d'ancrage **11,** notamment via la pièce **15,** et l'extrémité libre comprenant un butoir. Dans les deux cas, le butoir **131** peut être par exemple une boucle réalisée par la branche correspondante. De préférence, la boucle formant butoir présente un diamètre équivalent compris entre 1700 µm et 2200 µm.

La combinaison de branches de centrage longues et courtes **13, 12** permet à la fois de centrer le filtre par rapport à la paroi du vaisseau sanguin tout en limitant, voire empêchant, l'inclinaison de l'axe de celui-ci par rapport à l'axe local du vaisseau sanguin là où le filtre **1** est placé.

Toutes les branches **11, 12, 13** du filtre présentent avantageusement une section de diamètre équivalent compris entre 0,250 et 0,350 mm, de préférence entre 0,29 et 0,31 mm, toujours de préférence entre 0,295 et 0,305 mm. Dans le présent exposé, le terme « diamètre équivalent » de la section d'une branche désigne le diamètre du cercle le plus petit dans lequel peut s'inscrire la section de la branche. De préférence, toute branche du filtre présente une section de même diamètre équivalent sur toute sa longueur. De préférence encore, toutes des branches du filtre présentent une section de même diamètre équivalent. Avantageusement, la section de la branche est un disque.

De préférence, l'ensemble du filtre 1 est réalisé en acier inoxydable, de préférence INOX 316LVM.

De préférence, le filtre **1** présente une longueur longitudinale comprise entre 4,5 et 6,5 cm, toujours de préférence entre 5 et 6 cm, par exemple environ 5,5 cm. L'envergure des branches du filtre, c'est-à-dire le diamètre du cercle le plus petit dont le centre est aligné sur l'axe longitudinal du filtre et englobant l'ensemble des projections des branches sur un plan perpendiculaire à l'axe longitudinal du filtre et colinéaire à celui-ci, est de préférence compris entre 4 et 6 cm, toujours de préférence entre 4,5 et 5,5 cm, par exemple environ 5 cm.

La description donnée ci-dessus correspond au filtre lorsqu'il n'est pas contraint par un autre élément extérieur à celui-ci.

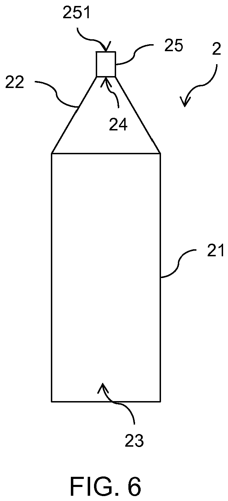

Lors de son insertion à l'intérieur du vaisseau sanguin, les branches **11, 12, 13** du filtre sont tout d'abord repliées, par exemple à l'aide d'un adaptateur **2.** Un exemple d'un tel adaptateur **2** (figure 6) comprend une partie cylindrique **21** à base circulaire creuse prolongée par une partie conique **22** creuse et tronquée à son sommet, présentant un plus grand orifice **23** du côté de la partie cylindrique et un plus petit orifice **24** du côté tronqué du cône. La surface intérieure de la partie cylindrique **21** est continue avec celle de la partie conique **22.** Le diamètre intérieur de la partie cylindrique **21** est suffisamment grand pour que le filtre **1** ne soit pas trop contraint, voire pas du tout. L'angle au sommet du cône est de préférence compris entre 50° et 60°, toujours de préférence entre 53° et 58°, par exemple 56°. Le cône est tronqué de manière à ce que le diamètre intérieur au niveau du plus petit orifice soit compris entre 2 mm et 3 mm, de préférence entre 2,4 et 2,6 mm. La combinaison du cône et du niveau de troncature permet le repliement des branches du filtre lorsque celui-ci est disposé à l'intérieur de l' adaptateur de manière à ce que les extrémités libres du filtre soient orientées vers le sommet du cône et lorsque le filtre est poussé à l'intérieur de l' adaptateur **2** à travers le plus petit orifice **24.** Le filtre peut alors être inséré à l'intérieur d'un cathéter. Pour cela, l' adaptateur **2** comprend également un connecteur **25** au niveau de la troncature du cône pour la connexion de l' adaptateur **2** à un cathéter. Le connecteur **25** comprend un orifice **251** dont le diamètre est au moins égal à celui du plus petit orifice **24** de l' adaptateur **2.** L'orifice **251** du connecteur est destiné à déboucher dans la lumière du cathéter, notamment un cathéter 7 F.

## Revendications

1. Filtre (1) pour veine cave, comprenant au moins deux branches d'ancrage (11) pour l'ancrage du filtre dans la paroi du vaisseau sanguin, les branches d'ancrage comportant une extrémité fixe (111) et une extrémité libre (112), les extrémités fixes des branches d'ancrage étant fixée l'une à l'autre, chacune des branches d'ancrage comprenant une protection anti-transfixion (114),
les extrémités libres étant terminées en crochet (113) orienté vers l'extérieur,
la protection anti-transfixion étant une vrille présentant un diamètre équivalent compris entre 990 et 1210 µm et la protection anti-transfixion étant réalisée sur la branche d'ancrage à proximité du crochet.

2. Filtre selon la revendication 1, dans lequel au moins deux branches d'ancrage présentent des longueurs différentes.

3. Filtre selon la revendication 1 ou la revendication 2, comprenant en outre trois branches de centrage courtes (12), chacune comprenant une extrémité fixe et une extrémité libre, l'extrémité fixe étant fixée aux extrémités fixes des branches d'ancrage, et l'extrémité libre étant atraumatique.

4. Filtre selon la revendication 3 dans lequel les trois branches de centrage courtes sont plus courtes que les branches d'ancrage.

5. Filtre selon la revendication 3 ou la revendication 4, dans lequel l'extrémité libre atraumatique présente un crochet (121) orienté vers l'intérieur.

6. Filtre selon l'une des revendications 3 à 5, dans lequel les branches de centrage courtes sont de longueur égale ou de longueurs différentes.

7. Filtre selon l'une des revendications 1 à 6, comprenant en outre trois branches de centrage longues (13), chacune présentant une extrémité fixe et une extrémité libre, l'extrémité fixe étant fixée aux extrémités fixes des branches d'ancrage.

8. Filtre selon la revendication 7, dans lequel les trois branches de centrage longues sont plus longues que les branches d'ancrage.

9. Filtre selon l'une des revendications 1 à 8, comprenant en outre une branche de contact présentant une extrémité fixe et une extrémité libre, l'extrémité fixe étant fixée aux extrémités fixes des branches d'ancrage et l'extrémité libre comprenant un butoir.

10. Filtre selon la revendication 8, dans lequel l'extrémité libre d'une des branches de centrage longues (13) comprend un butoir (131).

11. Filtre selon la revendication 9 ou la revendication 10, dans lequel le butoir est une boucle réalisée à partir de la branche correspondante.

12. Filtre selon l'une des revendications 1 à 11, entièrement réalisé en acier inoxydable, notamment INOX 316 LVM.

## Patentansprüche

1. Vena-cava-Filter (1), umfassend wenigstens zwei Verankerungsschenkel (11) zur Verankerung des Filters in der Wand des Blutgefäßes, wobei die Verankerungsschenkel ein festes Ende (111) und ein freies Ende (112) aufweisen, wobei die festen Enden der Verankerungsschenkel aneinander befestigt sind, wobei jeder der Verankerungsschenkel einen Durchbohrungsschutz (114) umfasst, wobei die freien Enden in einem nach außen gerichteten Haken (113) enden, wobei der Durchbohrungsschutz eine Windung mit einem äquivalenten Durchmesser zwischen 990 und 1210 µm ist und wobei der Durchbohrungsschutz auf dem Verankerungsschenkel in der Nähe des Hakens angebracht ist.

2. Filter nach Anspruch 1, wobei wenigstens zwei Verankerungsschenkel unterschiedliche Längen aufweisen.

3. Filter nach Anspruch 1 oder Anspruch 2, ferner umfassend drei kurze Zentrierschenkel (12), die jeweils ein festes und ein freies Ende aufweisen, wobei das feste Ende an den festen Enden der Verankerungsschenkel befestigt ist und das freie Ende atraumatisch ist.

4. Filter nach Anspruch 3, wobei die drei kurzen Zentrierschenkel kürzer sind als die Verankerungsschenkel.

5. Filter nach Anspruch 3 oder 4, wobei das freie atraumatische Ende einen nach innen gerichteten Haken (121) aufweist.

6. Filter nach einem der Ansprüche 3 bis 5, wobei die kurzen Zentrierschenkel gleich lang oder unterschiedlich lang sind.

7. Filter nach einem der Ansprüche 1 bis 6, ferner umfassend drei lange Zentrierschenkel (13), die jeweils ein festes und ein freies Ende aufweisen, wobei das feste Ende an den festen Enden der Verankerungsschenkel befestigt ist.

8. Filter nach Anspruch 7, wobei die drei langen Zentrierschenkel länger sind als die Verankerungsschenkel.

9. Filter nach einem der Ansprüche 1 bis 8, ferner umfassend einen Kontaktschenkel, der ein festes und ein freies Ende aufweist, wobei das feste Ende an den festen Enden der Verankerungsschenkel befestigt ist und das freie Ende einen Anschlag umfasst.

10. Filter nach Anspruch 8, wobei das freie Ende eines der langen Zentrierschenkel (13) einen Stopper (131) umfasst.

11. Filter nach Anspruch 9 oder 10, wobei der Stopper eine aus der entsprechenden Spule gefertigte Schlaufe ist.

12. Filter nach einem der Ansprüche 1 bis 11, der vollständig aus rostfreiem Stahl, insbesondere INOX 316 LVM, gefertigt ist.

## Claims

1. A vena cava filter (1), comprising at least two anchoring branches (11) for anchoring the filter in the wall of the blood vessel, the anchoring branches including a fixed end (111) and a free end (112), the fixed ends of the anchoring branches being attached to each other, each of the anchoring branches comprising an anti-transfixion protection (114),
the free ends terminating in a hook (113) oriented outwards,
the anti-transfixion protection being a loop having an equivalent diameter comprised between 990 and 1,210 µm and the anti-transfixion protection being made on the anchoring branch proximate to the hook.

2. The filter according to claim 1, wherein at least two anchoring branches have different lengths.

3. The filter according to claim 1 or claim 2, further comprising three short centring branches (12), each comprising a fixed end and a free end, the fixed end being attached to the fixed ends of the anchoring branches, and the free end being non-traumatic.

4. The filter according to claim 3, wherein the three short centring branches are shorter than the anchoring branches.

5. The filter according to claim 3 or claim 4, wherein the non-traumatic free end has a hook (121) oriented inwards.

6. The filter according to one of claims 3 to 5, wherein the short centring branches have equal lengths or different lengths.

7. The filter according to one of claims 1 to 6, further comprising three long centring branches (13), each having a fixed end and a free end, the fixed end being attached to the fixed ends of the anchoring branches.

8. The filter according to claim 7, wherein the three long centring branches are longer than the anchoring branches.

9. The filter according to one of claims 1 to 8, further comprising a contact branch having a fixed end and a free end, the fixed end being attached to the fixed ends of the anchoring branches and the free end comprising a stop.

10. The filter according to claim 8, wherein the free end of one of the long centring branches (13) comprises a stop (131).

11. The filter according to claim 9 or claim 10, wherein the stop is a loop made up from the corresponding branch.

12. The filter according to one of claims 1 to 11, entirely made of stainless steel, in particular STAINLESS STEEL 316 LVM.
